# EUROPÄISCHE PATENTSCHRIFT

(11) **EP 1 152 699 B1**
(45) Veröffentlichungstag und Bekanntmachung des Hinweises auf die Patenterteilung: **13.06.2007**
(21) Anmeldenummer: 00990775.9
(22) Anmeldetag: 15.12.2000
(51) Int. Cl.: A61B 17/22, A61M 1/00

(54) **MEDIZINISCHES INSTRUMENT ZUR ABTRAGUNG VON GEWEBE, KNOCHENZEMENT ODER DERGLEICHEN IM MENSCHLICHEN ODER TIERISCHEN KÖRPER**
MEDICAL INSTRUMENT FOR REMOVING TISSUE, BONE CEMENT AND THE LIKE IN THE HUMAN OR ANIMAL BODY
INSTRUMENT MEDICAL DESTINE A ENLEVER DES TISSUS, DU CIMENT OSSEUX ET ANALOGUES DU CORPS HUMAIN OU ANIMAL

(30) Priorität: 16.12.1999 DE 19961027
(43) Veröffentlichungstag der Anmeldung: 14.11.2001
(73) Patentinhaber: Karl Storz GmbH & Co. KG, 78532 Tuttlingen (DE)
(72) Erfinder: IRION, Klaus, 78576 Liptingen (DE); ISSE, Nicanor, G., Burbank, CA 91502 (US)
(74) Vertreter: Heuckeroth, Volker
(86) Internationale Anmeldenummer: PCT/EP2000/012829
(87) Internationale Veröffentlichungsnummer: WO 2001/043651

(56) Entgegenhaltungen:
- WO-A-99/09897
- DE-A- 3 439 434
- DE-A- 4 405 656
- DE-A- 19 817 979
- US-A- 4 886 491
- US-A- 5 685 840

## Beschreibung

Die Erfindung betrifft ein medizinisches Instrument zum Behandeln, insbesondere zum Abtragen von Gewebe, Knochenzement oder dergleichen im menschlichen oder tierischen Körper, mit einem Handstück und einem am distalen Ende desselben angeordneten Applikationsteil, wobei das Applikationsteil relativ zum Handstück axial hin- und herbewegbar ist, und wobei im Handstück ein Antriebsmechanismus für das Applikationsteil vorhanden ist, der einen im Handstück kontinuierlich anstehenden Druck in die Hin- und Herbewegung des Applikationsteils überführt, wobei der Antriebsmechanismus eine im Handstück angeordnete Druckkammer aufweist, in die zur Erzeugung des Drucks Druckluft von einer externen Druckluftquelle zugeführt wird, und die zumindest eine Entlüftungsöffnung zum Entweichen der Druckluft aufweist, und wobei in der Druckkammer ein mit dem Applikationsteil verbundener Kolben angeordnet ist, der durch ein Energiespeicherelement in Richtung entgegen dem Druck vorgespannt ist.

Ein derartiges Instrument ist aus der DE 34 39 434 A1 bekannt.

Das zuvor genannte bekannte Instrument dient der Erzeugung von mechanischen Schwingungen zu Heilzwecken auch in tieferer Körperschicht.

Ein Instrument der eingangs genannten Art soll gemäß der vorliegenden Erfindung dazu geeignet sein, biologisches Gewebe, vor allem Weich- oder Fettgewebe, im menschlichen oder tierischen Körper abzutragen. Ein spezieller Anwendungsfall eines solchen Instruments ist die Fettabsaugung im Rahmen der Schönheitschirurgie. Ein anderer Anwendungsfall eines derartigen Instruments ist die Abtragung von Knochenzement, mit dem eine Endoprothese, beispielsweise eine Hüftgelenksendoprothese, in einem Knochen des menschlichen Skeletts verankert ist, um die Endoprothese zum Zwecke einer Revision aus dem Knochen herausnehmen zu können.

Aus der US 5,685,840 ist ein Instrument mit einem Applikationsteil bekannt, das eine Öffnung am distalen Ende, sowie ein sich innerhalb des feststehenden Applikationsteils hin- und herbewegendes Schneidelement aufweist, das in die Öffnung des Applikationsteil eingesaugtes Gewebe im Zusammenwirken mit dem Rand der Öffnung abschneidet. Als Antrieb für das sich im Applikationsteil hin- und herbewegende Schneidelement ist ein Elektromotor vorgesehen, der im Handstück angeordnet ist.

Ein Nachteil bei diesem Instrument besteht darin, daß das abzutragende Gewebe zunächst in die Öffnung gesaugt und darauf folgend abgeschnitten wird, so daß es nicht vermieden werden kann, daß auch Gefäße in die Öffnung gesaugt und durchtrennt werden, was zu erheblichen Blutungen führen kann.

Ein weiterer Nachteil eines solchen Instruments besteht darin, daß aufgrund des im Handstück integrierten Motors das Instrument selbst ein hohes Gewicht besitzt, was zu einer raschen Ermüdung der Hand des Anwenders führt. Des weiteren ist ein Bewegungsumformelement erforderlich, um die Drehbewegung des Motors in eine axiale Bewegung des Schneidelements umzusetzen. Auch dieses Bewegungsumformelement ist relativ groß und schwer, und es treten Verluste bei der Umformung von der Drehbewegung in die axiale Bewegung des Schneidelements auf.

Für die Desintegration von Weichgewebe sind auch rein mechanische Instrumente bekannt, die in Form von Kanülen ausgebildet sind, die mit einer üblichen Spritze verbunden sind. Durch Hin- und Herbewegen der Kanüle von Hand kann Fettgewebe abgelöst und mittels der Spritze abgesaugt werden.

Der Nachteil bei diesen Instrumenten besteht darin, daß über die von Hand vermittelte mechanische Bewegung und die daraus resultierenden großen Hübe der Hin- und Herbewegung neben dem Fettgewebe auch Blutgefäße und anderes Gewebe abgelöst werden, was zu inneren Verletzungen und Entzündungen führen kann.

Weiterhin ist aus der US 4,886,491 ein Verfahren zur Gewebeabtragung mit einem Ultraschall-Instrument bekannt, bei der eine Ultraschall-Saugkanüle in den Körper in den Bereich des Weich- bzw. Fettgewebes eingeführt wird. Die Kanüle wird mittels Ultraschall zu hochfrequenten Schwingungen mit geringer Amplitude angeregt, wodurch eine lokale mechanische Gewebedesintegration, aber auch zusätzlich Reibungswärme erzeugt wird. Das desintegrierte Fettgewebe wird über die Kanüle dann abgesaugt.

Der Nachteil der ultraschallgestützten Gewebeabtragung besteht darin, daß es bei längeren Behandlungszeiten, wie dies insbesondere bei der Fettabsaugung im Abdominalbereich der Fall ist, die bis zu vier Stunden dauern kann, zu einer signifikanten Erwärmung des Ultraschallhandstücks kommt, die auch durch eine zusätzliche Spülung nicht verhindert werden kann. Darüber hinaus sind die Ultraschall-Handstücke mit den Piezoschwingern relativ groß und schwer. Dies ist insbesondere für die Fettabsaugung im Gesichtsbereich ein Problem. Darüber hinaus sind die Piezoelemente mit relativ hohen Spannungen zu betreiben, was zu einer zusätzlichen Isolations- bzw. Sicherheitsproblematik führt. Auch die Gewebeabtragungsgeschwindigkeit bzw. Desintegrationsgeschwindigkeit läßt zu wünschen übrig.

Demgegenüber wäre es auch denkbar, ein beispielsweise aus der WO 99/09897 bekanntes Instrument für die pneumatische Lithotripsie, das zur Fragmentierung harter Konkremente, wie Körpersteine, eingesetzt wird, in modifizierter Form auch für die Abtragung von Gewebe, insbesondere Weichgewebe und Fettgewebe, einzusetzen. Bei diesem bekannten Instrument für die pneumatische Lithotripsie wird ein in einer Sonde beweglicher Bolzen mittels Druckluftimpulsen axial hin- und herbewegt, wobei der durch den Bolzen vermittelte Sondenimpuls das aufzulösende Konkrement fragmentiert.

Ein weiterer derartiger Lithotripter ist aus der DE 44 05 656 A1 bekannt, bei dem die die Stoßwellen übertragende Sonde in einem feststehenden Führungsrohr verläuft, das einen An- und/oder Absaugkanal für die mit der Sonde zertrümmerten Steine aufweist.

Der Nachteil derartiger pneumatischer Lithotripsieinstrumente besteht in der geringen Wiederholfrequenz der Bewegung der Sonde, die weniger als 20 Hz beträgt, und in dem relativ großen Handstück. Die geringe Wiederholungsfrequenz der Sondenbewegung beruht darauf, daß Druckimpulse erzeugt werden müssen, und daß der Bolzen nach jedem Schuß in die Ausgangslage zurückbewegt werden muß. Außerdem ist neben dem Handstück und einer Druckluftversorgung ein zusätzliches Gerät für die Druckimpulserzeugung notwendig. Ein solches pneumatisches Lithotripsieinstrument, das für die Fragmentierung von Konkrementen aufgrund der sehr hohen Sondengeschwindigkeit bzw. des sehr starken Sondenimpulses optimiert ist, ist für die Abtragung von Gewebe oder Knochenzement unverhältnismäßig aufwendig.

Das aus der eingangs genannten DE 34 39 434 A1 bekannte Instrument weist als Applikationsteil eine Sonde, Kanüle oder Nadel auf, in deren distalem Teil ein Linearmotor vorhanden ist, der einen Kolben aufweist, der durch einen anstehenden Druck eines Fluides hin- und herbewegt wird, um Schwingungen bis etwa 20 kHz zu erzeugen, um verschiedene biologische Wirkungen an verschiedenen Stellen des Körpers, auch weit im Innern des Körpers, zu erreichen. Dieses bekannte Instrument eignet sich aufgrund der Ausgestaltung des Applikationsteils, das durch den Kolben selbst gebildet ist, nicht zur Abtragung von Weich- und Fettgewebe.

Der Erfindung liegt daher die Aufgabe zugrunde, ein Instrument der eingangs genannten Art bereitzustellen, mit dem Gewebe, insbesondere Weich- und Fettgewebe, ohne die Gefahr von Blutungen und inneren Verletzungen sowie Knochenzement oder dergleichen abgetragen werden kann, wobei die Behandlung an den jeweiligen Anwendungsfall anpaßbar sein soll.

Diese Aufgabe wird hinsichtlich des eingangs genannten medizinischen Instruments dadurch gelöst, daß das Applikationsteil zumindest im Bereich seines distalen Endes einen Kanal aufweist, der in seinem distalen Bereich zumindest eine Saug- und/oder Spülöffnung aufweist, und daß der Hub und/oder die Frequenz der Hin- und Herbewegung des Applikationsteils einstellbar ist, indem der wirksame Querschnitt der zumindest einen Entlüftungsöffnung verkleiner- und vergrößerbar ist.

Bei dem erfindungsgemäßen Instrument ist demnach das Applikationsteil selbst relativ zum Handstück axial hin- und herbewegbar, wodurch mit dem Applikationsteil in schonender Weise das abzutragende Gewebe gelockert wird. Insbesondere können Fettpartikel aus dem Gewebeverbund gelöst und durch die erfindungsgemäße Ausbildung des Applikationsteils als Hohlsonde abgesaugt werden und/oder es kann eine Spülflüssigkeit in das Behandlungsgebiet eingeleitet werden, um die Absaugung von Gewebe oder Knochenzement zu erleichtern.

Gegenüber den bekannten Instrumenten, bei denen das abzutrennende Gewebe durch eine Öffnung in das Applikationsteil gesaugt und mittels eines Schneidelements abgeschnitten wird, wird die Gefahr eines Ab- oder Zertrennens von Blutgefäßen und damit von Blutungen vermieden.

Gerade die Kombination aus dem durch den anstehenden Druck hin- und herbewegten Applikationsteils und dessen gleichzeitige Ausgestaltung als Hohlsonde führen zu einem konstruktiv einfachen Aufbau und zur besonderen Eignung des Instruments zum Abtragen von Weich- und Fettgewebe.

Während die bekannten Shaverinstrumente das abzutragende Gewebe ausschließlich aktiv schneiden, ermöglicht es das erfindungsgemäße Instrument im Unterschied dazu, Gewebeverbände mechanisch aufzulösen bzw. zu desintegrieren. Dadurch, daß als Antriebsmechanismus für das Applikationsteil ein kontinuierlich anstehender Druck ausgenutzt wird, der in eine Hin- und Herbewegung des Applikationsteils umgesetzt wird, wird gegenüber den aus der pneumatischen Lithotripsie bekannten Instrumenten der Vorteil geschaffen, daß die Wiederholfrequenz der Hin- und Herbewegung des Applikationsteils wesentlich erhöht werden kann. Es können Wiederholfrequenzen von mehr als 50 Hz erreicht werden. Gegenüber den rein mechanischen Instrumenten, die von Hand hin- und herbewegt werden, und bei denen somit der Bewegungshub groß und nicht genau kontrollierbar ist, kann der Bewegungshub des Applikationsteils aufgrund des Antriebsmechanismus je nach Anwendung auf etwa 0,1 mm bis 5 mm oder auch auf 10 mm begrenzt werden. Das erfindungsgemäße Instrument eignet sich nicht nur zur Abtragung von Gewebe oder Knochenzement, sondern es kann auch vorteilhaft zur Stimulation von Gewebebereichen durch Übertragung von Bewegungsenergie in den zu behandelnden Gewebebereich zur Therapie von Entzündungen oder Gelenkerkrankungen, Nervenentzündungen oder zur Verbesserung der Durchblutung eingesetzt werden.

Aufgrund des einfachen und kostengünstigen Aufbaus läßt sich das Instrument auch als Einweg-Instrument herstellen, so daß eine Reinigung und Sterilisation entfallen kann.

Der Druck ist durch Druckluft erzeugt und der Antriebsmechanismus ist in der Art eines pneumatisch-mechanischen Schwingkreises ausgebildet, der als Wirkelemente den kontinuierlich anstehenden pneumatischen Druck und zumindest ein Energiespeicherelement, vorzugsweise eine Feder, aufweist.

Durch diese Ausgestaltung wird ein konstruktiv einfacher, kostengünstiger Antriebsmechanismus für das Applikationsteil geschaffen, der den kontinuierlich anstehenden Druck mit einem Energiespeicherelement, insbesondere einer Feder, zu einem pneumatisch-mechanischen Schwingkreis vereint.

Dabei ist die Druckluft in einer externen Druckluftquelle erzeugt und wird dem Handstück über eine Leitung zugeführt.

Hierbei ist von Vorteil, daß das Handstück selbst schlank und gewichtsarm ausgebildet werden kann, weil die Druckluftquelle nicht im Handstück untergebracht werden muß. Als Druckluftquelle kann eine Druckluftflasche, ein lokales oder ein zentrales Kompressorsystem (krankenhausinterne Druckluftversorgung) verwendet werden.

Der Antriebsmechanismus weist eine im Handstück angeordnete Druckkammer auf, die eine Entlüftungsöffnung aufweist, wobei in der Druckkammer ein mit dem Applikationsteil verbundener Kolben angeordnet ist, der durch das Energiespeicherelement in Richtung entgegen dem Druck vorgespannt ist.

Durch diese Maßnahme wird ein konstruktiv besonders einfacher, leicht zu wartender pneumatisch-mechanischer Schwingkreis geschaffen, wobei die Schwingung des Antriebsmechanismus und damit die Hin- und Herbewegung des Applikationsteils durch das Zusammenwirken des Kolbens mit der zumindest einen seitlichen Entlüftungsöffnung hervorgerufen wird. Der kontinuierlich anstehende Druck bewegt den Kolben nach distal, wodurch die Feder gespannt wird, wobei der Kolben bei seiner Bewegung nach distal die zumindest eine Entlüftungsöffnung freigibt, aus der die Druckluft entweichen kann, wodurch sich der Druck in der Druckkammer abbaut, und die Feder den Kolben nach der Entlüftung wieder zurückbewegt, wonach sich der Druck in der Druckkammer von neuem aufbaut, so daß insgesamt eine periodische Bewegung des Applikationsteils hervorgerufen wird.

Der Hub und/oder die Frequenz der Hin- und Herbewegung des Applikationsteils ist einstellbar. Hierbei ist von Vorteil, daß das erfindungsgemäße Instrument auf die jeweilige Anwendung hinsichtlich des Bewegungshubs bzw. der Bewegungsamplitude angepaßt werden kann. Die Einstellbarkeit beläuft sich bevorzugt auf einen Bereich zwischen 0,1 mm und 5 mm oder größer, beispielsweise 10 mm. Zur Einstellung des Hubs und/oder der Frequenz ist dazu der wirksame Querschnitt der zumindest einen Entlüftungsöffnung verkleiner- und vergrößerbar.

Durch Vergrößern der Entlüftungsöffnung baut sich der Druck in der Druckkammer schneller ab, wenn der Kolben die Entlüftungsöffnung freigibt, wodurch die Bewegungsamplitude des Kolbens verringert wird, weil die im Energiespeicher bspw. in Form einer Feder gespeicherte Kraft als Gegenkraft zu dem anstehenden Druck aufgrund des rascheren Druckabfalls bereits nach kürzerer Zeit und damit kürzerem Bewegungsweg den Druck übersteigt und den Kolben wieder nach proximal zurückbewegt. Durch Verkleinern der Entlüftungsöffnung wird entsprechend der Bewegungshub des Kolbens bis zu seiner Bewegungsumkehr vergrößert.

Speziell kann durch eine axiale Veränderung des Wirkungsquerschnittes der zumindest einen Lüftungsöffnung im wesentlichen eine Änderung des Hubs des Applikationsteiles erreicht werden, während eine umfängliche Änderung des Wirkungsquerschnittes zu einem schnelleren Luftaustritt und damit zu einer anderen Frequenz der Hin- und Herbewegung führen kann.

Der Bewegungshub und/oder die Frequenz des Kolbens kann auf diese Weise durch die Regelung der Entlüftung eingestellt werden.

Dazu ist es weiterhin bevorzugt, wenn um die Druckkammer herum eine axial und/oder umfänglich bewegliche Hülse zum kontinuierlichen Abdecken bzw. Freilegen der Entlüftungsöffnung angeordnet ist.

In einer weiteren bevorzugten Ausgestaltung ist der Druck in der Druckkammer über ein einstellbares Druckminderventil einstellbar.

Hierbei ist von Vorteil, daß der in der Druckkammer anstehende kontinuierliche Druck im Verhältnis zum Energiespeicherelement und damit die Bewegungsgeschwindigkeit des Applikationsteils und der Hub der Bewegung des Applikationsteils eingestellt werden kann. Alternativ zu einem Druckminderventil kann auch der Wirkungsquerschnitt der Belüftungsöffnung, beispielsweise in Form einer Irisblende, variierbar sein, um den Befüllvorgang der Druckkammer zu steuern.

In bevorzugten Ausgestaltungen ist das Druckminderventil entweder im Handstück proximalseitig der Druckkammer angeordnet, oder in einem externen Gerät zwischen der Druckluftquelle und dem Handstück.

Weiterhin ist es auch bevorzugt, wenn das Druckminderventil unmittelbar an oder in der Druckluftquelle angeordnet ist.

Hierdurch kann die Einstellung des in der Druckkammer anstehenden Drucks unmittelbar an der Druckluftquelle eingestellt werden.

Weiterhin ist es bevorzugt, wenn die zumindest eine Saug- und/oder Spülöffnung seitlich am Applikationsteil angeordnet ist.

Aufgrund der seitlichen Anordnung der Öffnung wird eine Verletzungsgefahr durch die Ränder der Öffnung bei der axialen Hin- und Herbewegung vermieden, während dies bei einer Öffnung am distalen Ende des Applikationsteils nicht gewährleistet ist.

In einer weiteren bevorzugten Ausgestaltung ist eine distale Spitze des Applikationsteiles geschlossen und etwa kugelkappenförmig ausgebildet.

Während sich diese Ausgestaltung des distalen Endes des Applikationsteiles vorteilhafterweise zur Desintegration von Weich- oder Fettgewebe eignet, weil eine schonendere Behandlung des Gewebes gewährleistet ist, ist es gemäß einer weiteren Ausgestaltung ebenso bevorzugt, wenn das Applikationsteil eine distal offene Spitze aufweist, die die Saug- und Spülöffnung bildet.

In diesem Fall ergibt sich eine hohlmeißelähnliche Ausgestaltung des Applikationsteiles, mit dem Knochenzement in Verbindung mit der Hin- um Herbewegung des Applikationsteiles besonders effizient abgetragen werden kann. Dabei können die Ränder der distal offenen Spitze zusätzlich geschärft sein.

In weiteren bevorzugten Ausgestaltungen ist im proximalen Bereich des Kanals distalseitig des Handstücks ein Anschlußstutzen zum Anschließen einer Spül- und/oder Saugleitung angeordnet, oder der Kanal reicht bis in das Handstück hinein und mündet am proximalen Ende des Handstücks in einen Anschlußstutzen zum Anschließen einer Saug- und/oder Spülleitung.

Bei letzterer Alternative ergibt sich der Vorteil eines geraden Kanals zur verbesserten Absaugung von abgetragenem Gewebe, und ein weiterer Vorteil besteht in einer geringeren Störbeeinflussung des Anwenders durch etwaige Schläuche im Bereich des Applikationsteils.

Weiterhin ist es bevorzugt, wenn das Applikationsteil zumindest einen Kanal zur Insufflation eines Fluids in das Behandlungsgebiet aufweist.

Bei dieser Ausgestaltung kann durch Insufflieren eines geeigneten Fluids die Desintegration des Gewebes vorteilhaft beschleunigt werden, wodurch die Behandlungsdauer insbesondere bei der Fettabsaugung verringert werden kann.

Außerdem ist es bevorzugt, wenn die zumindest eine Saug- und/oder Spülöffnung an ihrem Rand als Schneide ausgebildet ist.

Durch diese Maßnahme kann die Gewebeabtragung durch die Kombination aus mechanischer Desintegration und zusätzlicher Schneidwirkung des Randes der Spülöffnung beim Hin- und Herbewegen des Applikationsteils verbessert werden, wobei im Unterschied zu den bekannten Instrumenten, bei denen das Gewebe ausschließlich in die Öffnung gesaugt und mittels eines Schneidelements abgetrennt wird, hierbei eine Gefahr der Verletzung von Blutgefäßen verringert ist.

Um eine zusätzliche elektrisch bedingte Gewebedesintegration mitbewirken zu können, ist es ebenso bevorzugt, wenn das Applikationsteil zumindest eine elektrisch isolierte Elektrode aufweist, die mit einer Hochfrequenzspannung beaufschlagbar ist. Im Falle nur einer Elektrode kann monopolar, im Falle von zwei Elektroden bipolar desintegriert werden.

Auch kann die Gewebedesintegration vorteilhaft verbessert werden, wenn an das Applikationsteil zusätzlich ein Ultraschall erzeugendes Geberelement ankoppelbar ist.

In einer weiteren bevorzugten Ausgestaltung ist das Applikationsteil vom Handstück abnehmbar.

Hierdurch läßt sich das Instrument aufgrund der Zerlegbarkeit in Handstück und Applikationsteil leichter und gründlicher reinigen.

Eine noch weitere Verbesserung des erfindungsgemäßen Instruments wird in einer bevorzugten Ausgestaltung dadurch erreicht, daß in dem Applikationsteil ein bild- und/oder lichtübertragendes endoskopisches System angeordnet ist, wodurch die Anwendung des Instruments im Behandlungsbereich endoskopisch visualisiert und damit kontrolliert werden kann.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: ein medizinisches Instrument zum Behandeln, insbesondere Abtragen von Gewebe, Knochenzement oder dgl. im menschlichen oder tierischen Körper in einer Längsschnittdarstellung in einer ersten momentanen Betriebsstellung des Applikationsteils;
- Fig. 2: das Instrument in Fig. 1 in einer zweiten momentanen Betriebsstellung des Applikationsteils;
- Fig. 3: eine Gesamtvorrichtung zum Behandeln, insbesondere Abtragen von Gewebe, Knochenzement oder dgl. im menschlichen oder tierischen Körper mit dem Instrument in Fig. 1 und 2;
- Fig. 4: das distale Ende des Applikationsteiles des Instrumentes in vergrößerter perspektivischer Darstellung; und
- Fig. 5: ein alternatives Ausführungsbeispiel für das distale Ende des Applikationsteiles.

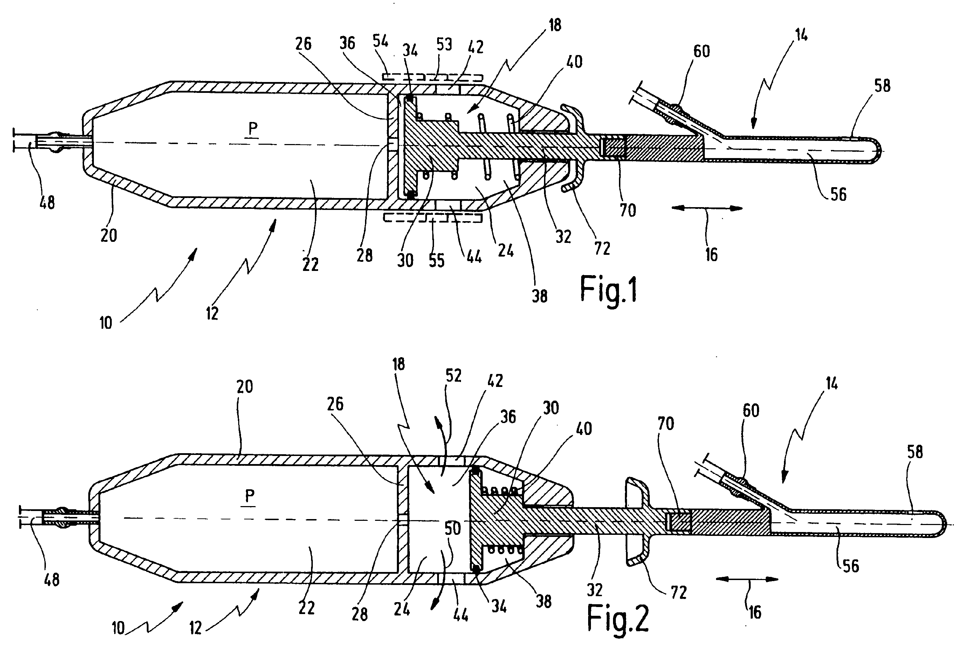

In Fig. 1 bis 3 ist ein mit dem allgemeinen Bezugszeichen 10 versehenes medizinisches Instrument dargestellt, mit dem Gewebe im menschlichen oder tierischen Körper behandelt werden kann. Das Instrument 10 dient insbesondere zum Abtragen von Gewebe, insbesondere Weich- oder Fettgewebe, oder zum Abtragen von Knochenzement, mit dem eine Endoprothese in einem Knochen des Skeletts implantiert ist. Das Instrument 10 kann außerdem zur Stimulation von Gewebebereichen durch Übertragung von Bewegungsenergie in den zu behandelnden Gewebebereich zur Therapie von Entzündungen, Gelenkerkrankungen, Nervenentzündungen oder zur Verbesserung der Durchblutung von Gewebe verwendet werden.

Das Instrument 10 weist ein Handstück 12 und ein am distalen Ende des Handstücks 12 angeordnetes Applikationsteil 14 auf.

Das Applikationsteil 14 ist relativ zum Handstück 12 axial hin- und herbewegbar, wie mit einem Doppelpfeil 16 angedeutet ist.

Um das Applikationsteil 14 gemäß dem Doppelpfeil 16 hin- und herzubewegen, ist im Handstück 12 ein Antriebsmechanismus 18 für das Applikationsteil 14 vorhanden, der einen kontinuierlich anstehenden Druck in die Hin- und Herbewegung des Applikationsteils 14 überführt, wie hiernach noch näher beschrieben wird.

Das Handstück 12 weist ein Gehäuse 20 auf, das das Handstück 12 in eine proximale Kammer 22, in der der mit p bezeichnete Druck kontinuierlich ansteht und in eine distale Druckkammer 24 unterteilt.

Die proximale Kammer und die Druckkammer 24 sind durch eine Trennwand 26 voneinander getrennt, wobei in der Trennwand 26 eine Belüftungsöffnung 28 ausgespart ist. Die Belüftungsöffnung 28 ist mittig in der Trennwand 26 angeordnet und geht durch die Trennwand 26 hindurch.

In der Druckkammer 24 ist ein Kolben 30 angeordnet, der mit dem Applikationsteil 14 über ein axiales Verlängerungsstück 32 verbunden ist, wobei das Verlängerungsstück 32 vom Applikationsteil 14 aus gesehen in das Gehäuse 20 des Handstücks 12 hineinragt.

Der Kolben 30 weist eine Dichtung 34 in Form eines O-Rings auf, wodurch die Druckkammer 24 in eine proximale Teilkammer 36 und eine distale Teilkammer 38 unterteilt ist, wobei die proximale Teilkammer 36 durch die Dichtung 34 gegen die distale Teilkammer 38 druckdicht abgedichtet ist. Mit anderen Worten wird nur die proximale Teilkammer 36 mit dem kontinuierlich anstehenden Druck p über die Belüftungsöffnung 28 beaufschlagt.

Der Kolben 30 ist weiterhin mit einem in der Druckkammer 24 angeordneten Energiespeicherelement 40 in Form einer Druckfeder nach proximal vorgespannt. Das Energiespeicherelement 40 wirkt somit dem kontinuierlich anstehenden Druck p entgegen.

In dem die Druckkammer 24 umgebenden Teil des Gehäuses 20 sind zwei seitliche Entlüftungsöffnungen 42 und 44 ausgespart, die sich in Umfangsrichtung nur über einen Teilumfang der Druckkammer 24 erstrecken.

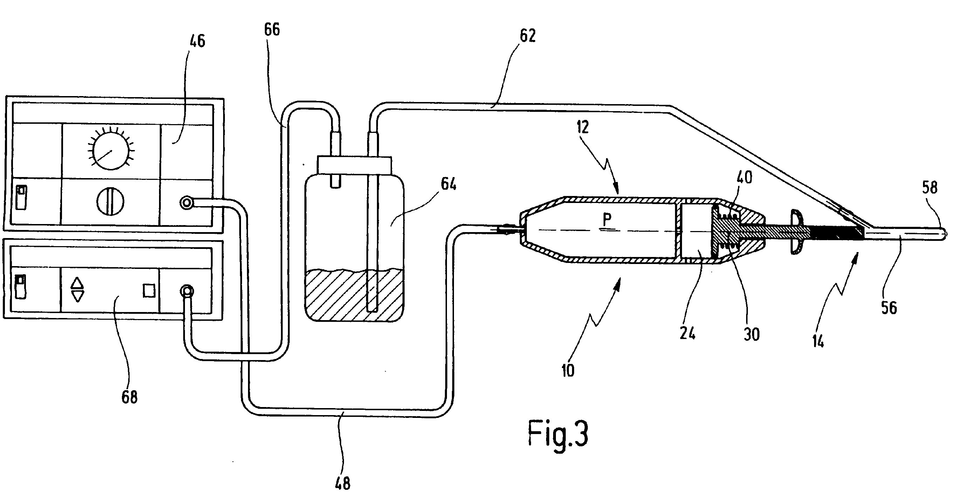

Der Druck p wird durch Druckluft erzeugt, die gemäß Fig. 3 in einer externen Druckluftquelle 46 kontinuierlich erzeugt und über eine Leitung 48 in die proximale Kammer 22 des Handstücks 12 zugeführt wird, um dort den kontinuierlichen Druck p zu erzeugen.

Mit den zuvor beschriebenen Elementen des durch die Druckluft erzeugten Druckes p und dem Energiespeicherelement 40 in Form der Feder wird ein pneumatisch-mechanischer Schwingkreis gebildet, der zu der periodischen Hin- und Herbewegung des Applikationsteils 14 führt, wie im folgenden beschrieben wird.

Ausgehend von der in Fig. 1 dargestellten Stellung des axial in der Druckkammer 24 angeordneten Kolbens, in der sich die Dichtung 34 proximalseitig der Entlüftungsöffnungen 42 und 44 befindet, bewirkt der in der proximalen Kammer 22 und durch die Belüftungsöffnung 28 auch in der proximalen Teilkammer 36 der Druckkammer 24 anstehende kontinuierliche Druck, daß der Kolben 30 entgegen der Kraft des Energiespeicherelements 40 nach distal gedrückt wird. Sobald der Kolben 30 durch die Belüftung der proximalen Teilkammer 36 der Druckkammer 24 so weit nach distal bewegt wurde, daß die Dichtung 34 distalseitig der Entlüftungsöffnungen 42 und 44 zu liegen kommt, wird die proximale Teilkammer 36 der Druckkammer 24 durch Entweichen der Druckluft aus den Entlüftungsöffnungen 42 und 44 entlüftet, wie mit Pfeilen 50 und 52 angedeutet ist. Dadurch baut sich der Druck in der proximalen Teilkammer 36 der Druckkammer 24 ab, wodurch das gespannte Energiespeicherelement 40 den Kolben 30 wieder aus der in Fig. 2 dargestellten Stellung in die in Fig. 1 dargestellte Stellung zurückbewegt, von der aus sich der Vorgang wiederholt. Dies führt zu der periodischen Hin- und Herbewegung des Applikationsteils 14 gemäß dem Doppelpfeil 16. Die Stellungen des Kolbens 30 in Fig. 1 und Fig. 2 sind als Momentaufnahmen der periodischen Bewegung zu verstehen.

Der Bewegungshub des Applikationsteils 14 wird durch den kontinuierlich anstehenden Druck p, die Federkonstante des Energiespeicherelementes 40 und die Wirkungsquerschnitte und Position der Entlüftungsöffnungen 42 und 44 und der Belüftungsöffnung 28 bestimmt.

Zur Einstellung des kontinuierlich anstehenden Druckes p ist ein nicht dargestelltes einstellbares Druckminderventil vorgesehen, das bspw. im Handstück angeordnet sein kann, und zwar proximalseitig der proximalen Kammer 22, oder das in einem nicht dargestellten externen Gerät zwischen der Druckluftquelle 46 und dem Handstück 12 oder auch an bzw. in der Druckluftquelle 46 selbst angeordnet sein und über ein entsprechendes Betätigungselement eingestellt werden kann. Der Belüftungsvorgang der Druckkammer 24 mit Luft kann auch dadurch steuerbar sein, daß die Belüftungsöffnung im Querschnitt verkleiner- oder vergrößerbar ist, beispielsweise kann die Belüftungsöffnung in Form einer Irisblende ausgestaltet sein.

In Fig. 1 ist mit unterbrochenen Linien weiterhin eine axial und/oder umfänglich beweglich am Gehäuse 20 angeordnete und entsprechend abgedichtete Hülse 54 schematisch dargestellt, mit der der Hub und die Frequenz der Hin- und Herbewegung des Applikationsteils 14 ebenfalls einstellbar sind, indem die Hülse 54 mit entsprechenden Öffnungen 53 und 55 versehen ist, die je nach ihrer axialen oder umfänglichen Stellung mit den Entlüftungsöffnungen 42 und 44 nicht, teilweise oder ganz fluchten, wodurch die Wirkungsquerschnitte der Entlüftungsöffnungen 42 und 44 verkleinert oder vergrößert werden, so daß die Druckluft aus den Entlüftungsöffnungen 42 bzw. 44 schneller oder langsamer bzw. früher oder später entweicht. Gibt die Hülse 54 beispielsweise einen größeren Wirkungsquerschnitt der Entlüftungsöffnungen 42 bzw. 44 frei, kann die Druckluft schneller aus diesen Entlüftungsöffnungen 42 und 44 entweichen, wodurch sich der Druck p in der proximalen Teilkammer 36 der Druckkammer 24 schneller abbaut, wodurch sich der distale Umkehrpunkt der Bewegung des Kolbens 30 weiter nach proximal verlagert. Bei einem verringerten Wirkungsquerschnitt der Entlüftungsöffnungen 42 bzw. 44 wird der distale Umkehrpunkt der Bewegung des Kolbens 30 weiter nach distal verlagert.

Der Hub der Hin- und Herbewegung des Applikationsteils 14 kann auf diese Weise in einem Bereich zwischen 0,1 mm und 5 mm oder bis 10 mm variiert werden.

Die Wiederholfrequenz der periodischen Hin- und Herbewegung, die mit diesem pneumatisch-mechanischen Schwingkreis erreicht werden kann, liegt bei mehr als 50 Hz.

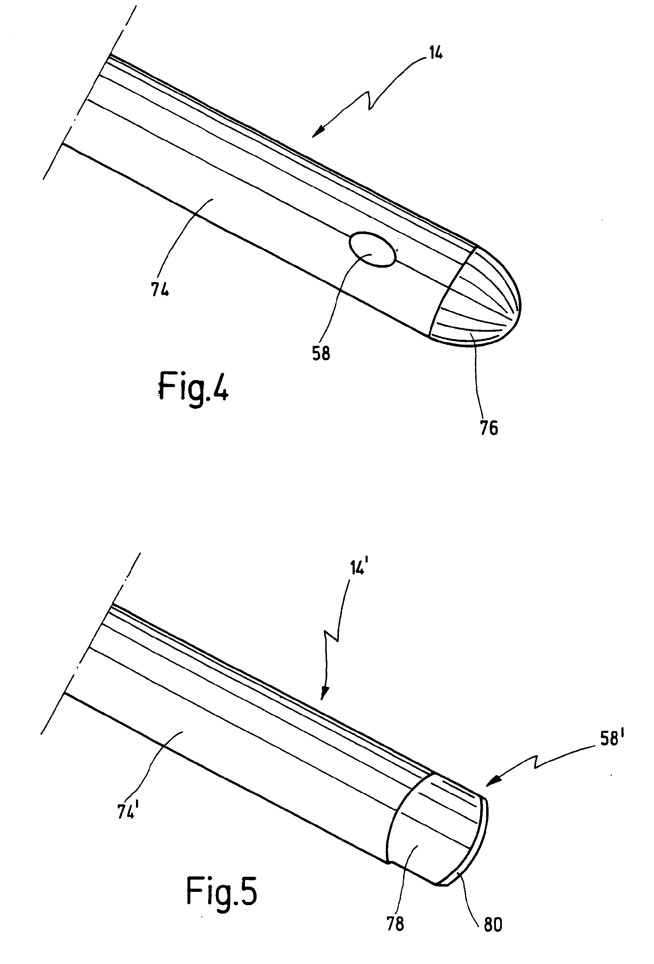

Im folgenden wird die Ausgestaltung des Applikationsteils 14 näher beschrieben, dessen distales Ende vergrößert in Fig. 4 dargestellt ist.

Das Applikationsteil 14 weist im Bereich seines distalen Endes einen Kanal 56 auf, so daß das Applikationsteil 14 im Bereich seines distalen Endes als Hohlsonde ausgebildet ist. Der Kanal 56 weist eine seitlich angeordnete Saug- und/oder Spülöffnung 58 auf. Gemäß Fig. 4 weist das Applikationsteil 14 ein zylindrisches Rohr 74 mit einer Spitze 76 auf, die etwa kugelkappenförmig ausgebildet ist. Bei speziellen Applikatoren wie beispielsweise für die Katarakt-Chirurgie bzw. für die Entnahme des Linsenkörpers im Auge kann auch eine frontale Öffnung von Vorteil sein.

Im proximalen Bereich des Kanals 56 ist noch distalseitig des Handstücks 12 ein Anschlußstutzen 60 zum Anschließen einer Saug- und/oder Spülleitung 62 angeordnet, wie in Fig. 3 dargestellt ist. In dem gezeigten Ausführungsbeispiel ist die Saug- und/oder Spülleitung 62 lediglich als Saugleitung ausgebildet, die in ein Unterdruckbehältnis 64 geführt ist. Über die Saugleitung 62 kann mit dem Applikationsteil 14 desintegriertes abgetragenes Gewebe in das Unterdruckbehältnis 64 gesaugt werden. Um den zum Ansaugen erforderlichen Unterdruck in dem Unterdruckbehältnis 64 zu erzeugen, ist das Unterdruckbehältnis 64 über eine Leitung 66 mit einer Saugpumpe 68 verbunden.

Der Kanal 56 in dem Applikationsteil 14 selbst oder ein nicht dargestellter weiterer Kanal im Applikationsteil 14 kann zur Insufflation eines Fluids in das Behandlungsgebiet dienen.

Des weiteren kann der Rand der seitlichen Saug- und Spülöffnung 58 als Schneide ausgebildet sein, um aufgrund der Hin- und Herbewegung des Applikationsteils 14 zusätzlich eine mechanische Schneidwirkung zum Abtragen von Gewebe bzw. zum Desintegrieren von Gewebe zu erzeugen. Eine weitere Ausbildung besteht darin, daß in der Öffnung eine Art Gitter eingelegt ist, über das ein Vielfachschneiden bzw. Raspeln möglich ist.

Das Applikationsteil 14 selbst ist von dem Handstück 12 abnehmbar, indem das Applikationsteil 14 über eine lösbare Verbindung 70, bspw. in Form einer Verschraubung, mit dem Kolben 30, genauer gesagt mit dem axialen Verlängerungsstück 32 des Kolbens 30, verbunden ist.

An dem axialen Verlängerungsstück 32 des Kolbens 30 ist ferner ein Luftabweiser 72 vorgesehen, um zu verhindern, daß durch die Entlüftungsöffnungen 42 bzw. 44 entweichende unsterile Druckluft in das Behandlungsgebiet geblasen wird.

Das Instrument 10 eignet sich insbesondere im Rahmen der minimal-invasiven Chirurgie zur desintegrierenden Abtragung von Fettgewebe, wozu das Applikationsteil 14 durch eine kleine Inzision eingeführt wird. Nach Einschalten der Druckluftquelle 46 führt das Applikationsteil 14 wie zuvor beschrieben eine periodische Hin- und Herbewegung aus, die auf mechanischem Wege zur Zerstörung von Zellverbänden und damit zur Desintegration des Fettgewebes führt. Bei eingeschalteter Saugpumpe 68 wird das desintegrierte Fettgewebe gleichzeitig durch die Saug- und/oder Spülöffnung 58 in den Kanal 56 gesaugt und über den Anschlußstutzen 60 und die Leitung 62 in das Unterdruckbehältnis 64 abgeführt.

In einer weiteren nicht dargestellten Weiterbildung kann das Applikationsteil zumindest proximalseitig von einer Außenhülle beispielsweise in Form einer Einführhilfe umgeben sein, so daß die Einstichstelle nicht direkt dem sich bewegenden Applikationsteil ausgesetzt ist.

In weiteren nicht dargestellten Weiterbildungen des Applikationsteils 14 kann dieses zumindest eine elektrisch isolierte Elektrode aufweisen, die mit einer Hochfrequenzspannung beaufschlagbar ist, so daß zusätzlich zur mechanischen Gewebeabtragung aufgrund der periodischen Hin- und Herbewegung des Applikationsteils 14 die desintegrierende Wirkung durch Hochfrequenzstrom verstärkt werden kann.

Außerdem kann an das Applikationsteil 14 ein Ultraschallerzeugungselement ankoppelbar sein, so daß zusätzlich der Hin- und Herbewegung des Applikationsteils 14 noch eine hochfrequente Vibration überlagert werden kann, wodurch die Desintegration ebenfalls verstärkt werden kann.

Um im Rahmen der minimal-invasiven Chirurgie eine Sichtkontrolle der Behandlung zu-ermöglichen, kann im Applikationsteil 14 weiterhin ein bild- und/oder lichtübertragendes endoskopisches System angeordnet sein.

Auch ist es möglich, den Kanal 56 des Applikationsteils 14 in das Handstück 12 bis zu dessen proximalem Ende ragen zu lassen, und den Anschlußstutzen 60 dann am proximalen Ende des Handstücks 12 anzuordnen.

In Fig. 5 ist in einem weiteren Ausführungsbeispiel ein distales Ende eines Applikationsteiles 14' dargestellt, das mit dem Instrument 10 anstelle des Applikationsteiles 14 verbunden werden kann.

Das Applikationsteil 14' weist ein zylindrisches Rohr 74' auf, dessen distales Ende eine als Saug- und Spülöffnung 58' dienende offene Spitze 78 aufweist. Die Spitze 78 weist einen geschärften Rand auf.

Diese Ausgestaltung des Applikationsteiles 14' in Form eines Hohlmeißels eignet sich besonders zur Abtragung von Knochenzement.

## Patentansprüche

1. Medizinisches Instrument zum Behandeln, insbesondere zum Abtragen, von Gewebe, Knochenzement oder dergleichen im menschlichen oder tierischen Körper, mit einem Handstück (12) und einem am distalen Ende desselben angeordneten Applikationsteil (14; 14'), wobei das Applikationsteil (14; 14') relativ zum Handstück (12) axial hin- und herbewegbar ist, wobei im Handstück ein Antriebsmechanismus (18) für das Applikationsteil (14; 14') vorhanden ist, der einen im Handstück kontinuierlich anstehenden Druck (p) in die Hin- und Herbewegung des Applikationsteils (14; 14') überführt, wobei der Antriebsmechanismus (18) eine im Handstück (12) angeordnete Druckkammer (24) aufweist, in die zur Erzeugung des Drucks (p) Druckluft von einer externen Druckluftquelle (46) zugeführt wird, und die zumindest eine Entlüftungsöffnung (42, 44) zum Entweichen der Druckluft aufweist, und wobei in der Druckkammer (24) ein mit dem Applikationsteil (14; 14') verbundener Kolben (30) angeordnet ist, der durch ein Energiespeicherelement (40) in Richtung entgegen dem Druck (p) vorgespannt ist, **dadurch gekennzeichnet, daß** das Applikationsteil (14; 14') zumindest im Bereich seines distalen Endes einen Kanal (56) aufweist, der in seinem distalen Bereich zumindest eine Saug- und/oder Spülöffnung (58; 58') aufweist, und daß der Hub und/oder die Frequenz der Hin- und Herbewegung des Applikationsteils (14; 14') einstellbar ist, indem der wirksame Querschnitt der zumindest einen Entlüftungsöffnung (42, 44) verkleiner- und vergrößerbar ist.

2. Instrument nach Anspruch 1, **dadurch gekennzeichnet, daß** der Druck (p) in der Druckkammer (24) über ein einstellbares Druckminderventil einstellbar ist.

3. Instrument nach Anspruch 2, **dadurch gekennzeichnet, daß** das Druckminderventil im Handstück (12) proximalseitig der Druckkammer (24) angeordnet ist.

4. Instrument nach Anspruch 2, **dadurch gekennzeichnet, daß** das Druckminderventil in einem externen Gerät zwischen der Druckluftquelle (46) und dem Handstück (12) angeordnet ist.

5. Instrument nach Anspruch 4, **dadurch gekennzeichnet, daß** das Druckminderventil unmittelbar an oder in der Druckluftquelle (46) angeordnet ist.

6. Instrument nach einem der Ansprüche 1 bis 5, **dadurch gekennzeichnet, daß** die zumindest eine Saug- und/oder Spülöffnung (58) seitlich am Applikationsteil (14) angeordnet ist.

7. Instrument nach einem der Ansprüche 1 bis 6, **dadurch gekennzeichnet, daß** eine distale Spitze (76) des Applikationsteiles (14) geschlossen und etwa kugelkappenförmig ausgebildet ist.

8. Instrument nach einem der Ansprüche 1 bis 7, **dadurch gekennzeichnet, daß** das Applikationsteil (14') eine distal offene Spitze (78) aufweist, die die Saug- und Spülöffnung (58') bildet.

9. Instrument nach einem der Ansprüche 1 bis 8, **dadurch gekennzeichnet, daß** im proximalen Bereich des Kanals (56) distalseitig des Handstücks (12) ein Anschlußstutzen (60) zum Anschließen einer Saug- und/oder Spülleitung (62) angeordnet ist.

10. Instrument nach einem der Ansprüche 1 bis 9, **dadurch gekennzeichnet, daß** der Kanal bis in das Handstück (12) hineinreicht und am proximalen Ende des Handstücks (12) in einen Anschlußstutzen zum Anschließen einer Saug- und/oder Spülleitung mündet.

11. Instrument nach einem der Ansprüche 1 bis 10, **dadurch gekennzeichnet, daß** das Applikationsteil (14; 14') zumindest einen Kanal zur Insufflation eines Fluids in das Behandlungsgebiet aufweist.

12. Instrument nach einem der Ansprüche 1 bis 11, **dadurch gekennzeichnet, daß** die zumindest eine Saug- und/oder Spülöffnung (58) an ihrem Rand als Schneide ausgebildet ist.

13. Instrument nach einem der Ansprüche 1 bis 12, **dadurch gekennzeichnet, daß** um die Druckkammer (24) herum eine axial und/oder umfänglich bewegliche Hülse (54) zum kontinuierlichen Abdecken bzw. Freilegen der zumindest einen Entlüftungsöffnung (42, 44) angeordnet ist.

14. Instrument nach einem der Ansprüche 1 bis 13, **dadurch gekennzeichnet, daß** das Applikationsteil (14) zumindest eine elektrisch isolierte Elektrode aufweist, die mit einer Hochfrequenzspannung beaufschlagbar ist.

15. Instrument nach einem der Ansprüche 1 bis 14, **dadurch gekennzeichnet, daß** zusätzlich ein Ultraschall erzeugendes Geberelement an das Applikationsteil (14) ankoppelbar ist.

16. Instrument nach einem der Ansprüche 1 bis 15, **dadurch gekennzeichnet, daß** das Applikationsteil (14; 14') vom Handstück (12) abnehmbar ist.

17. Instrument nach einem der Ansprüche 1 bis 16, **dadurch gekennzeichnet, daß** im Applikationsteil (14) ein bild- und/oder lichtübertragendes endoskopisches System angeordnet ist.

## Claims

1. A medical instrument for treating, in particular for removing tissue, bone cement or the like in the human or animal body, comprising a hand piece (12) and an application part (14; 14') arranged on the distal end of same, wherein the application part (14; 14') is axially movable to-and-fro relative to the hand piece (12), wherein the hand piece contains a drive mechanism (18) for the application part (14; 14'), which transfers a pressure (p) being continuously present in the hand piece into the to-and-fro movement of the application part (14; 14'), wherein the drive mechanism (18) has a pressure chamber (24) arranged in the hand piece (12), into which, for creating the pressure (p), compressed-air is supplied from an external compressed air source (46), and which has at least one air outlet opening (42, 44) to let the compressed air escape, and wherein in the pressure chamber (24) a piston (30) connected to the application part (14; 14') is arranged, which is biased by an energy storing element (40) in the direction against the pressure (p), **characterized in that** the application part (14; 14') has at least in the region of its distal end a conduit (56), which has in its distal region at least a suction and/or irrigation aperture (58; 58'), and that the length of stroke and/or the frequency of the to-and-fro movement of the application part (14; 14') is adjustable by the fact that the effective cross section of the at least one air outlet opening (42, 44) can be reduced and enlarged.

2. The instrument of claim 1, **characterized in that** the pressure (p) in the pressure chamber (24) is adjustable via an adjustable pressure reducing valve.

3. The instrument of claim 2, **characterized in that** the pressure reducing valve in the hand piece (12) is arranged on the proximal side of the pressure chamber (24).

4. The instrument of claim 2, **characterized in that** the pressure reducing valve is arranged in an external device between the compressed-air source (46) and the hand piece (12).

5. The instrument of claim 4, **characterized in that** the pressure reducing valve is arranged directly on or in the compressed-air source (46).

6. The instrument of anyone of claims 1 through 5, **characterized in that** the at least one suction and/or irrigation aperture (58) is arranged laterally on the application part (14).

7. The instrument of anyone of claims 1 through 6, **characterized in that** a distal point (76) of the application part (14) is closed and configured approximately in spherical cap shaped fashion.

8. The instrument of anyone of claims 1 through 7, **characterized in that** the application part (14') has a distally open point (78), which forms the suction and irrigation aperture (58').

9. The instrument of anyone of claims 1 through 8, **characterized in that** in the proximal region of the conduit (56), a connection piece (60) for connecting a suction and/or irrigation line (62) is arranged on the distal side of the hand piece (12).

10. The instrument of anyone of claims 1 through 9, **characterized in that** the conduit extends until into the hand piece (12) and opens into a connection piece (60) at the proximal end of the hand piece (12) for connecting a suction and/or irrigation line.

11. The instrument of anyone of claims 1 through 10, **characterized in that** the application part (14; 14') has at least one conduit for insufflation of a fluid into the treatment area.

12. The instrument of anyone of claims 1 through 11, **characterized in that** the at least one suction and/or irrigation aperture (58) is configured as a blade on its edge.

13. The instrument of anyone of claims 1 through 12, **characterized in that** around the pressure chamber (24) an axially and/or circumferentially movable sleeve (54) is arranged for continuous covering or exposing the at least one air outlet opening (42, 44).

14. The instrument of anyone of claims 1 through 13, **characterized in that** the application part (14) has at least one electrically insulated electrode, which can be supplied with a high frequency voltage.

15. The instrument of anyone of claims 1 through 14, **characterized in that** additionally a generator element generating ultrasound can be coupled onto the application part (14).

16. The instrument of anyone of claims 1 through 15, **characterized in that** the application part (14; 14') can be detached from the hand piece (12).

17. The instrument of anyone of claims 1 through 16, **characterized in that** in the application part (14) an endoscopic system is arranged, which transmits images and/or light.

## Revendications

1. Instrument médical pour le traitement, en particulier pour l'enlèvement, de tissu, de ciment d'os ou similaires dans le corps humain ou le corps animal, comprenant une poignée (12) et une partie d'application (14 ; 14') disposée sur l'extrémité distale de celle-ci, la partie d'application (14 ; 14') pouvant être déplacée axialement d'un côté et de l'autre par rapport à la poignée (12), un mécanisme d'entraînement (18) pour la partie d'application (14 ; 14') étant présent dans la poignée, lequel mécanisme convertit une pression (p) s'appliquant de façon continue dans la poignée en mouvement aller et retour de la partie d'application (14 ; 14'), le mécanisme d'entraînement (18) présentant une chambre de pression (24) disposée dans la poignée (12), dans laquelle de l'air comprimé provenant d'une source d'air comprimé (46) externe est amené pour générer la pression (p), et qui présente au moins une ouverture de purge d'air (42, 44) pour l'échappement de l'air comprimé, et un piston (30) relié à la partie d'application (14 ; 14') étant disposé dans la chambre de pression (24), lequel piston est pré-tendu par un élément d'accumulateur d'énergie (40) dans la direction contraire à la pression (p), **caractérisé en ce que** la partie d'application (14 ;14') présente au moins dans la zone de son extrémité distale un conduit (56) qui présente dans sa zone distale au moins une ouverture d'aspiration et/ou de lavage (58 ; 58'), et **en ce que** la course et/ou la fréquence du mouvement aller et retour de la partie d'application (14 ; 14') est réglable en réduisant et en augmentant la section efficace de la au moins une ouverture de purge d'air (42 ; 44).

2. Instrument selon la revendication 1, **caractérisé en ce que** la pression (p) dans la chambre de pression (24) est réglable au moyen d'une vanne réductrice de tension réglable.

3. Instrument selon la revendication 2, **caractérisé en ce que** la vanne réductrice de pression est disposée dans la poignée (12) côté proximal de la chambre de pression (24).

4. Instrument selon la revendication 2, **caractérisé en ce que** la vanne réductrice de pression est disposée dans un appareil externe entre la source d'air comprimé (46) et la poignée (12).

5. Instrument selon la revendication 4, **caractérisé en ce que** la vanne réductrice de pression est disposée directement sur ou dans la source d'air comprimé (46).

6. Instrument selon l'une quelconque des revendications 1 à 5, **caractérisé en ce que** la au moins une ouverture d'aspiration et/ou de lavage (58) est disposée sur le côté de la partie d'application (14).

7. Instrument selon l'une quelconque des revendications 1 à 6, **caractérisé en ce qu'**une pointe (76) distale de la partie d'application est fermée et conçue à peu près en forme de capuchon sphérique.

8. Instrument selon l'une quelconque des revendications 1 à 7, **caractérisé en ce que** la partie d'application (14') présente une pointe (78) ouverte côté distal qui forme l'ouverture d'aspiration et de lavage (58').

9. Instrument selon l'une quelconque des revendications 1 à 8, **caractérisé en ce qu'**une tubulure de raccordement (60) pour le raccordement d'une conduite d'aspiration et/ou de lavage (62) est disposée dans la zone proximale du conduit (56) côté distal de la poignée (12).

10. Instrument selon l'une quelconque des revendications 1 à 9, **caractérisé en ce que** le conduit va jusque dans la poignée (12) et débouche sur l'extrémité optimale de la poignée (12) dans une zone de tubulure pour le raccordement d'une conduite d'aspiration et/ou de lavage.

11. Instrument selon l'une quelconque des revendications 1 à 10, **caractérisé en ce que** la partie d'application (14 ; 14') présente au moins un conduit pour l'insufflation d'un fluide dans la zone de traitement.

12. Instrument selon l'une quelconque des revendications 1 à 11, **caractérisé en ce que** la au moins une ouverture d'aspiration et/ou de lavage (58) est conçue sur son bord comme lame.

13. Instrument selon l'une quelconque des revendications 1 à 12, **caractérisé en ce qu'**autour de la chambre de pression (24) est disposée une douille (54) mobile axialement et/ou sur le pourtour pour le recouvrement continu ou la libération de la au moins une ouverture de purge (42, 44).

14. Instrument selon l'une quelconque des revendications 1 à 13, **caractérisé en ce que** la partie d'application (14) présente au moins une électrode isolée électriquement qui peut être sollicitée avec une tension de haute fréquence.

15. Instrument selon l'une quelconque des revendications 1 à 14, **caractérisé en ce qu'**en supplément un élément transmetteur générant des ultrasons peut être couplé à la partie d'application (14).

16. Instrument selon l'une quelconque des revendications 1 à 15, **caractérisé en ce que** la partie d'application (14 ; 14') peut être enlevée de la poignée (12).

17. Instrument selon l'une quelconque des revendications 1 à 16, **caractérisé en ce qu'**un système endoscopique transmettant des images et/ou de la lumière est disposé dans la partie d'application (14).
